# EUROPÄISCHE PATENTSCHRIFT

(11) **EP 2 218 518 B1**
(45) Veröffentlichungstag und Bekanntmachung des Hinweises auf die Patenterteilung: **21.09.2011**
(21) Anmeldenummer: 10152760.4
(22) Anmeldetag: 05.02.2010
(51) Int. Cl.: B05C 17/005, B65D 81/32

(54) **Mehrkomponentenkartusche zur einmaligen Verwendung**
Multicomponent cartridge for one-time use
Cartouche à plusieurs composants à usage unique

(30) Priorität: 13.02.2009 EP 09152854
(43) Veröffentlichungstag der Anmeldung: 18.08.2010
(73) Patentinhaber: Sulzer Mixpac AG, 9469 Haag (CH)
(72) Erfinder: Staub, Andreas, 8404, Winterthur (CH); Leue, Percy, 78224, Singen (DE)
(74) Vertreter: Sulzer Management AG

(56) Entgegenhaltungen:
- WO-A1-2005/118154
- WO-A1-2009/021033
- DE-U1- 20 316 879
- DE-U1-202008 007 801

## Beschreibung

Die Erfindung betrifft eine Mehrkomponentenkartusche zur einmaligen Verwendung, welche zum gleichzeitigen Austrag von zwei Komponenten geeignet ist, welche vor der Verwendung gemischt werden können.

Eine derartige Mehrkomponentenkartusche ist bereits aus der DE 20 2008 007 801 U1 bekannt.

Nachteilig an diesem Aufbau ist, dass eine grosse Anzahl an Einzelteilen verwendet werden muss. Die Mehrkomponentenkartusche nach dem Stand der Technik ist sowohl für die einmalige Verwendung, als auch für eine mehrfache Verwendung konzipiert. Allerdings hat sich gezeigt, dass bei einer mehrfachen Verwendung einer derartigen Mehrkomponentenkartusche die Durchmischung ungleichmässig sein kann, sowie eine derartige Mehrkomponentenkartusche schwierig abzudichten sein kann, nachdem sie zum ersten Mal verwendet worden ist. Daher sind einmal benutzte Mehrkomponentenkartuschen nur begrenzt lagerfähig. Die Füllmasse kommt durch die mangelnde Abdichtung in Kontakt mit Luft und kann sich dadurch in ihren Eigenschaften ändern, also beispielsweise aushärten.

Eine Mehrkomponentenkartusche in koaxialer Bauweise, welche auch in der DE 20 2008 007 801 U1 gezeigt ist, kann weniger Probleme bei der Abdichtung aufweisen, allerdings bleibt die Problematik der ungleichmässigen Durchmischung bestehen. Die ungleichmässige Durchmischung hat zur Folge, dass bei Wiederverwendung der Mehrkomponentenkartusche jedes Mal, wenn die Mehrkomponentenkartusche benutzt wird, das Resultat der Durchmischung unterschiedlich ist, das heisst eine konstante Qualität der Füllmasse nicht erreicht werden kann.

Aufgabe der Erfindung ist daher eine Mehrkomponentenkartusche zu entwickeln, welche eine einfachere Bauweise hat und ausschliesslich zur einmaligen Verwendung bestimmt ist.

Eine weitere Aufgabe der Erfindung besteht darin, eine unbeabsichtigte Bewegung des Kolbens zu durch versehentliche Betätigung des Stössels verhindern.

Eine weitere Aufgabe der Erfindung besteht darin, dem Benutzer optisch anzuzeigen, ob eine befüllte Mehrkomponentenkartusche unversehrt ist.

Die Lösung umfasst eine Mehrkomponentenkartusche, welche eine erste Vorratskammer für eine erste Komponente, eine zweite Vorratskammer für eine zweite Komponente umfasst. Die erste Komponente ist im Lagerzustand getrennt von der zweiten Komponente. Die erste Vorratskammer ist koaxial um die zweite Vorratskammer herum angeordnet und bildet einen Ringraum aus, wobei in der ersten Vorratskammer ein erster Kolben beweglich aufgenommen ist und in der zweiten Vorratskammer ein zweiter Kolben beweglich aufgenommen ist. Der erste und zweite Kolben sind mittels eines Stössels bewegbar, um die beiden Komponenten gleichzeitig auszutragen. Ein Führungselement ist vorgesehen, um den ersten Kolben in der ersten Vorratskammer zu führen und um den zweiten Kolben in der zweiten Vorratskammer zu führen. Das Führungselement enthält eine Austrittsöffnung, durch welche die erste Komponente aus der ersten Vorratskammer austreten kann. Das Führungselement ist in einem Gehäuse angeordnet, wobei das Führungselement relativ zu dem Gehäuse mittels eines Bewegungselements bewegbar ist, wodurch die Austrittsöffnung freigebbar ist.

Das Bedienkonzept der erfindungsgemässen Mehrkomponentenkartusche unterscheidet sich somit grundlegend vom Stand der Technik. Der Anwender, welcher eine bestimmte Menge Füllmasse benötigt, nimmt eine Mehrkomponentenkartusche, wie vorher beschrieben zur Hand. Als erstes versichert er sich, dass die Mehrkomponentenkartusche unversehrt ist. Hierzu bewegt er das Bewegungselement auf die auf dem Gehäuse der Kartusche angezeigte Weise. Lässt sich diese Bewegung des Bewegungselements nicht ausführen, weiss der Anwender, dass die Mehrkomponentenkartusche bereits geöffnet worden ist.

Kann der Anwender das Bewegungselement in der vorgesehenen Weise betätigen, öffnet er hiermit die Austrittsöffnungen, sodass die Füllmasse durch die Austrittsöffnungen hindurchtreten kann und zum Mischer gefördert werden kann. Der Anwender kann die Mehrkomponentenkartusche nach seinen Wünschen ausrichten, um die Füllmasse an den gewünschten Ort zu bringen. Hierzu kann er die Mehrkomponentenkartusche auch in ein handelsübliches Austraggerät einbauen. Das Austraggerät umfasst ein Druckmittel, welches einen Druck auf den Stössel der Mehrkomponentenkartusche ausübt, welcher die Kolben in ihren Vorratskammern in Bewegung versetzt, wodurch die Füllmasse aus den entsprechenden Vorratskammern ausgeschoben wird. Durch die Austrittsöffnungen gelangt die Füllmasse zum Mischer, wird gemischt und tritt am Ende des Mischers aus. Das Ende des Mischers kann geeignete Elemente zur Positionierung des Strahls der Mischung der Komponenten der Füllmasse enthalten.

Vorteilhafterweise umfasst das Führungselement den Mischer, insbesondere einen statischen Mischer. Die Position des Führungselements relativ zum Mischer ist durch diese Massnahme vorgegeben. Das bedeutet aber, dass für alle Mehrkomponentenkartuschen dieser Bauweise zu erwarten ist, dass die Durchmischung von gleicher Qualität ist. Das Führungselement enthält auch die Austrittsöffnungen, sodass der Strömungsverlauf für die Komponenten für jede einzelne Mehrkomponentenkartusche gleich ist. Somit kann überraschenderweise mit dem erfindungsgemässen Konzept eine bessere Reproduzierbarkeit in bezug auf die erhaltene Mischung erreicht werden, obwohl für jede Charge eine eigene Mehrkomponentenkartusche benötigt wird. Zudem ergibt sich durch diese Lösung eine geringere Anzahl an Einzelteilen, sodass der Zusammenbau der Mehrkomponentenkartusche sehr einfach erfolgen kann.

Für die Befüllung und den Zusammenbau der Mehrkomponentenkartusche sind somit keine komplizierten Montageschritte erforderlich. Hieraus ergibt sich, dass die Befüllung kurz vor dem beabsichtigten Einsatz erfolgen kann, da die Befüllung dezentral an verschiedenen Orten vorgenommen werden kann. Dieser Vorteil fällt umsomehr ins Gewicht, wenn die Füllmasse nur sehr begrenzte zeitliche Haltbarkeit aufweist.

Das Bewegungselement umfasst ein an dem Führungselement angebrachtes Aussengewinde, in welches ein am Gehäuse angebrachtes Innengewinde eingreifen kann. Diese Ausführungsform des Bewegungselements als Drehelement ist bevorzugt, da sie einfach zu handhaben ist und da ein definierter Drehwinkel einer definierten Spaltbreite zwischen dem zweiten Endbereich des Führungselements und dem Gehäuse zuzuordnen ist.

Nach einem zweiten Ausführungsbeispiel können der Kolben und der Stössel einstückig ausgebildet sein. Diese einstückige Bauweise ist vorteilhaft, da neben der Verringerung der Summe der Bauteile und der damit verbundenen Vereinfachung der Mehrkomponentenkartusche auch eine Fehlpositionierung eines der Kolben und somit eine Schrägstellung der Kolben gänzlich ausgeschlossen sind. Der Kolbenteil des Stössels kann demzufolge auch eine geringere Bauhöhe aufweisen. Der Kolbenteil wird somit durch das Verbindungselement eines Gehäuseelements geführt, sodass ein Verkanten des Kolbenteils gemäss dieses Ausführungsbeispiels vermieden werden kann.

Der Stössel kann bevorzugt mit dem Gehäuseelement einstückig verbunden sein. Das Gehäuseelement mit dem Stössel wird nach Befüllung der Vorratskammern mit den entsprechenden Komponenten und dem Einsetzen der ersten und zweiten Kolben auf die Kolben aufgesetzt und mit dem Führungselement verbunden. Hierzu findet sich am Gehäuseelement ein Eingriffselement, welches mit einem Federelement am Führungselement zum Eingriff gebracht wird. Das Gehäuseelement ist somit mit dem Führungselement drehfest verbunden. Der mit dem Gehäuseelement verbundene Stössel hält die Kolben in ihrer Ausgangsposition, sodass die Füllmasse in den Vorratskammern eingeschlossen ist. In diesem Zustand kann die befüllte Mehrkomponentenkartusche gelagert werden, der Zustand wird nachfolgend als Lagerzustand bezeichnet. Wenn Stössel, Kolben und Gehäuseelement eine Einheit bilden, wird diese Einheit nach dem Befüllen mit dem Führungselement verbunden.

Das Gehäuseelement weist eine Sollbruchstelle auf, über welche der Stössel mit dem Gehäuseelement im Lagerzustand verbunden ist. Die Sollbruchstelle kann als Dichtung wirken, um den Innenraum des Gehäuseelements frei von Verunreinigungen zu halten. Nach einem weiteren Ausführungsbeispiel umfasst die Sollbruchstelle Rippen oder Stege, die in Längsrichtung bezogen auf die Stösselachse verlaufen. Des weiteren ermöglicht sie dem Anwender eine Beurteilung, ob die Mehrkomponentenkartusche unversehrt ist. Ist die Sollbruchstelle intakt, muss beim Austrag ein erhöhter Widerstand zu Beginn des Austragvorgangs überwunden werden, der dadurch bedingt ist, dass zum Durchbruch der Sollbruchstelle ein erhöhter Kraftaufwand erforderlich ist. Der Durchbruch der Sollbruchstelle ist sichtbar an der beginnenden Verschiebung des Stössel relativ zum Gehäuseelement sowie zumeist hörbar.

Der Stössel ist dann relativ zum Gehäuseelement bewegbar, wenn der Stössel mit einer Kraft beaufschlagt wird, wenn ein Austrag der ersten und zweiten Komponente erfolgen soll, wobei die Verbindung zwischen Gehäuseelement und Stössel unterbrochen wird.

Das Führungselement ist über das Eingriffselement mit einem Gehäuseelement verbindbar. Die Verwendung des Eingriffselements erlaubt eine einfache und problemlose Montage der Mehrkomponentenkartusche nach Befüllung der Vorratskammern. Insbesondere kann das Eingriffselement ein Federelement umfassen, wobei das Federelement als Vorsprung am Umfang des Führungselements ausgebildet sein kann. Das Federelement greift in eine Ausnehmung des Gehäuseelements ein, sodass das Gehäuseelement drehfest mit dem Führungselement verbunden ist.

Der erste Kolben umfasst einen Ringkolben, welcher eine ringförmige Dichtung an seinem äusseren Kolbenmantel aufweist. Die erste Vorratskammer ist koaxial zur zweiten Vorratskammer angeordnet, da diese Anordnung platzsparend ist und ein kleines Bauvolumen der Mehrkomponentenkartusche erzielt wird. Da die erste Vorratskammer ringförmig ist, ist der erste Kolben als Ringkolben ausgestaltet. Selbstverständlich könnte die erste Vorratskammer auch einen eckigen Querschnitt aufweisen. Der erste Kolben kann immer noch ringförmig sein, allerdings ist seine Form nicht mehr kreisringförmig.

Der zweite Kolben weist bevorzugt ein Entlüftungselement im Bereich der Sollbruchstelle auf, da beim Einsetzen des Kolbens nach dem Befüllen der Vorratskammern mit den entsprechenden Komponenten Luft zwischen der Füllmasse und dem Kolben verbleiben kann, die den Austragvorgang nachteilig beeinflussen kann. Alternativ oder in Ergänzung dazu kann das Führungselement ein Entlüftungselement umfassen.

Der Stössel und der Kolben können zumindest teilweise hohl sein. Hierdurch wird der Materialverbrauch für den Stössel und die Kolben verringert. Des weiteren können Stössel und Kolben einfacher im Spritzgiessverfahren hergestellt werden, wenn Materialanhäufungen vermieden werden und dünnwandige Bauteile verwendet werden können. Jedes der Bauteile, welche die Mehrkomponentenkartusche ausbilden kann als aus zumindest teilweise geschäumtem Kunststoff bestehen.

Die Verwendung eines einstückigen Kolbens, der gleichzeitig als Stössel wirkt, hat folgende Vorteile:

Die Mehrkomponentenkartusche kann in ein Standard-Austraggerät eingesetzt werden, welches am Markt weit verbreitet ist. Der Endanwender muss somit kein zusätzliches Austraggerät erwerben, sondern kann die Mehrkomponentenkartusche mit einem Standard-Austraggerät einsetzen.

Die Verbindung zum Standard-Austraggerät bildet der Stössel. Dieser Stössel kann eine für das Standard-Austraggerät geeignete Abmessung aufweisen.

Die Mehrkomponentenkartusche ist nur für eine einmalige Anwendung einsetzbar. Sie ist nicht für eine mehrfache Benutzung geeignet. Daher ist auch der statische Mischer nicht austauschbar.

Die Mehrkomponentenkartusche kann in schlanker und schmaler Bauweise ausgeführt werden. Daher kann die Mehrkomponentenkartusche einfacher gelagert und transportiert werden.

Es ist möglich, die Mehrkomponentenkartusche erst kurz vor der Anwendung zu befüllen. Daher können die leeren Mehrkomponentenkartuschen bedenkenlos gelagert werden und das Füllmaterial einfacher in geeigneten Behältern getrennt von den Kartuschen gelagert werden.

Des weiteren können die Mehrkomponentenkartuschen bzw. ihre Einzelteile einfacher und kostengünstiger im leeren Zustand transportiert werden.

Zudem kann die Mehrkomponentenkartusche über einen Schutz gegen unbeabsichtigtes Öffnen verfügen. Der Stössel kann hierzu mit dem ihn umgebenden Gehäuse fest verbunden sein. Erst beim Austrag wird die Verbindung zwischen Stössel und Gehäuse durch den auf den Stössel aufgebrachten Druck unterbrochen. Somit ist zu jeder Zeit sichtbar, ob die Mehrkomponentenkartusche noch neu ist oder sich bereits in Gebrauch befunden hat, also nicht mehr unversehrt ist. Zudem entsteht beim Durchtrennen der Verbindung zwischen Gehäuse und Stössel ein Geräusch, sodass auch akustisch erkennbar ist, ob die Mehrkomponentenkartusche vor Gebrauch unversehrt, also neuwertig war. Eine nicht autorisierte Wiederbefüllung oder Wiederverwendung kann auf einfache Weise vermieden werden.

Das erfindungsgemässe Konzept weist weniger Einzelteile als der Stand der Technik auf. Weil beispielsweise der Mischer mit dem Gehäuse der Kartusche fest verbunden ist, ist die Anströmung des Mischers während dem gesamten Austragzyklus gleich. Hieraus folgt nicht nur, dass die Mischgüte ein und derselben Mehrkomponente für den gesamten Austragzyklus im wesentlichen gleich ist, sondern es auch zu geringeren Abweichungen in der Mischgüte bei verschiedenen Mehrkomponentenkartuschen gleichen Typs kommt.

Nachfolgend wird die Erfindung anhand der Zeichnungen erläutert. Es zeigen:
- Fig. 1: einen Schnitt durch eine Mehrkomponentenkartusche gemäss eines ersten Ausführungsbeispiels der Erfindung
- Fig. 2: ein Detail des Kolbens der Mehrkomponentenkartusche gemäss Fig.1
- Fig. 3: ein Detail der Mehrkomponentenkartusche gemäss Fig. 1 im Bereich der ersten Austrittsöffnung
- Fig. 4: die Mehrkomponentenkartusche gemäss Fig. 1 in der Austragstellung.
- Fig. 5: ein Detail der Mehrkomponentenkartusche gemäss Fig. 1 im Bereich der ersten Austrittsöffnung in der Austragstellung
- Fig. 6: ein zweites Ausführungsbeispiel einer Mehrkomponentenkartusche gemäss der Erfindung
- Fig. 7: ein Detail des Kolbens der Mehrkomponentenkartusche gemäss Fig. 6
- Fig. 8: die Mehrkomponentenkartusche gemäss Fig. 6 in der Austragstellung.
- Fig. 9: ein Detail der Mehrkomponentenkartusche gemäss Fig. 6 im Bereich der Austrittsöffnung in Austragstellung
- Fig. 10: eine Aussenansicht der Mehrkomponentenkartusche gemäss eines der vorherigen Ausführungsbeispiele
- Fig. 11: einen Schnitt durch eine weitere Variante der Mehrkomponentenkartusche.
- Fig. 12: einen weiteren Schnitt durch die Variante der Mehrkomponentenkartusche gemäss Fig. 11.
- Fig. 13: ein Detail der Mehrkomponentenkartusche nach Fig. 11 oder Fig. 12

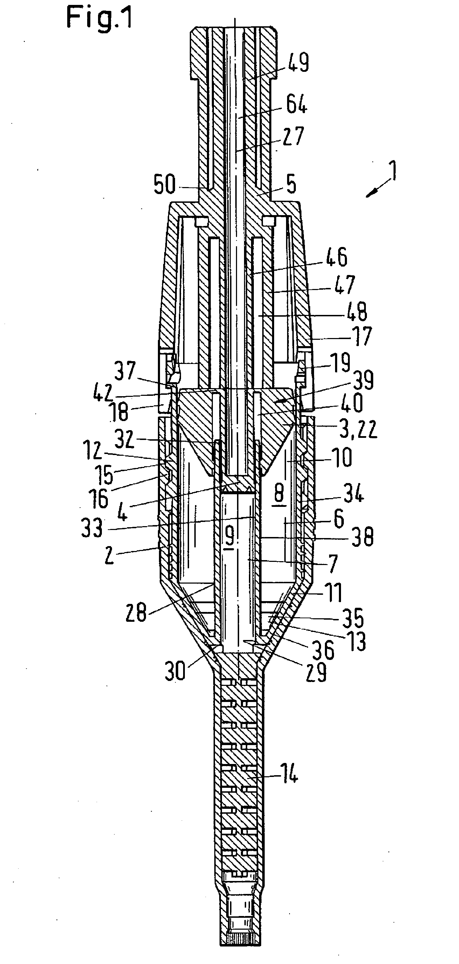

Fig. 1 zeigt ein erstes Ausführungsbeispiel der erfindungsgemässen Mehrkomponentenkartusche 1, welche zur einmaligen Verwendung bestimmt ist. Insbesondere wird eine derartige Mehrkomponentenkartusche zur Dosierung kleiner und kleinster Mengen an Füllmasse verwendet. Die Mehrkomponentenkartusche 1 umfasst eine erste Vorratskammer 6 für eine erste Komponente 8, eine zweite Vorratskammer 7 für eine zweite Komponente 9. Die erste Vorratskammer 6 ist getrennt von der zweiten Vorratskammer 7, sodass die beiden Komponenten nicht miteinander in Kontakt kommen. Derartige Komponenten interagieren zumeist miteinander, sobald sie miteinander in Berührung kommen, wobei chemische Reaktionen ablaufen können. Die Interaktion der Komponenten ist zumeist der Effekt, welcher in einer Anwendung benötigt wird, allerdings ist diese Interaktion unerwünscht, solange die Komponenten nicht im Rahmen der für sie bestimmten Anwendung eingesetzt werden. Vor dem Gebrauch der Mehrkomponentenkartusche muss sie somit gelagert und transportiert werden, und zwar teilweise in befülltem Zustand, der nachfolgend als Lagerzustand bezeichnet wird. Für die gesamte Dauer des Lagerzustandes muss sichergestellt sein, dass die beiden Komponenten 8,9 nicht miteinander in Berührung kommen.

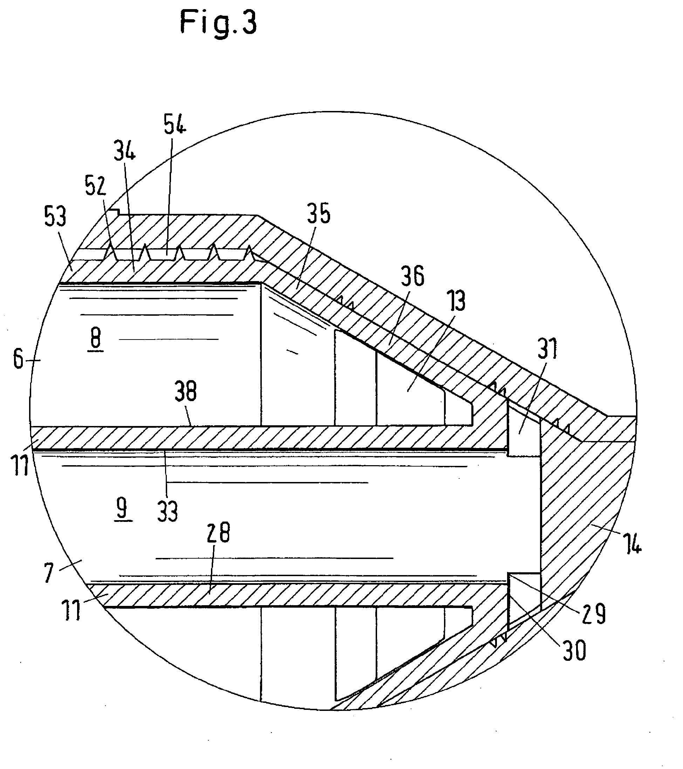

Die erste Vorratskammer 6 ist koaxial um die zweite Vorratskammer 7 herum angeordnet und bildet einen Ringraum 10 aus. Der Ringraum kann kreisringförmig ausgebildet sein. Die erste Vorratskammer 6 ist von der zweiten Vorratskammer 7 durch eine Trennwand 28 getrennt, sodass die beiden Komponenten 8,9 getrennt gelagert werden können. In diesem Ausführungsbeispiel erstreckt sich die zweite Vorratskammer 7 entlang einer Längsachse, welche mit der Längsachse 27 der Mehrkomponentenkartusche zusammenfällt. Die Trennwand 28 bildet die äussere Begrenzung der zweiten Vorratskammer 7 und umgibt die Vorratskammer 7 als Mantel. Die Trennwand 28 mündet an einem ersten Ende 30 in eine zweite Austrittsöffnung 29. Durch die zweite Austrittsöffnung 29 kann die zweite Komponente 9 zum Mischer 14 geführt werden, siehe auch Fig. 3. Es können auch mehrere zweite Austrittsöffnungen 29 vorgesehen sein, zwischen denen Stege 31 angeordnet sind, welche die Verbindung zum Mischer 14 ausbilden.

Die Trennwand 28 ist ein Teil des Führungselements 11. Die Trennwand 28 hat ein zweites Ende 32, welches zur Aufnahme eines zweiten Kolbens 4 dient. In der zweiten Vorratskammer 7 ist der zweite Kolben 4 beweglich aufgenommen. Dieser zweite Kolben 4 gleitet entlang einer Innenseite 33 der Trennwand 28 des Führungselements 11 in Richtung des ersten Endes 30, wenn die in der zweiten Vorratskammer 7 befindliche Füllmasse, also die zweite Komponente 9 ausgeschoben werden soll. Das Führungselement 11 ist vorgesehen, um den zweiten Kolben 4 in der zweiten Vorratskammer 7 zu führen.

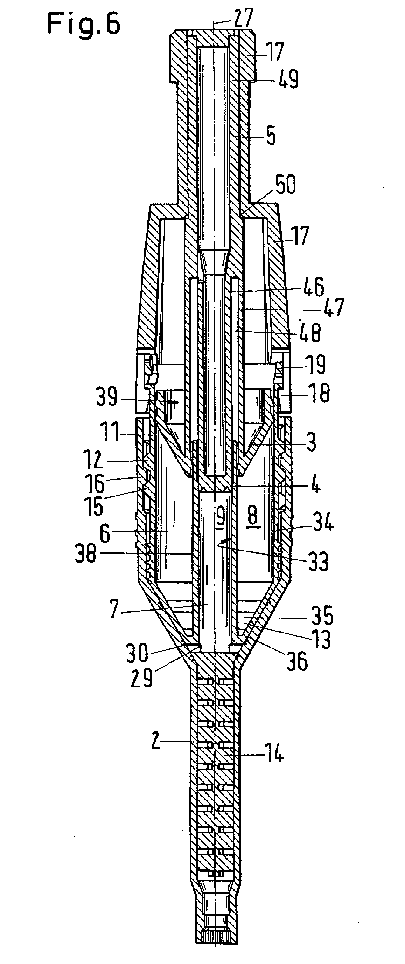

In der ersten Vorratskammer 6 ist ein erster Kolben 3 beweglich aufgenommen. Das Führungselement 11 ist vorgesehen, um den ersten Kolben 3 in der ersten Vorratskammer 6 zu führen. Die erste Vorratskammer 6 wird an ihrer Innenseite von der Trennwand 28 begrenzt und an ihrer Aussenseite von einem Mantelelement 34 des Führungselements 11 umgeben. Das Mantelelement 34 mündet an einem ersten Endbereich 35 in eine erste Austrittsöffnung 13. Durch die erste Austrittsöffnung 13 kann die erste Komponente 8 zum Mischer 14 geführt werden, siehe auch Fig. 3 oder Fig. 6. Es können auch mehrere erste Austrittsöffnungen 13 vorgesehen sein, zwischen denen Verbindungsstege 36 angeordnet sind, welche die Verbindung zur Trennwand 28 oder zum Mischer 14 ausbilden.

Das Mantelelement 34 ist ein Teil des Führungselements 11. Die Trennwand 28 und das Mantelelement 34 haben einen Endbereich 35, welcher zur Aufnahme eines ersten Kolbens 3 dient. In der ersten Vorratskammer 6 ist der erste Kolben 3 beweglich zwischen dem Mantelelement 34 und der Aussenseite 38 der Trennwand 28 aufgenommen. Dieser erste Kolben 3 gleitet entlang der Aussenseite 38 der Trennwand 28 des Führungselements 11 in Richtung des Endbereichs 35, wenn die in der ersten Vorratskammer 6 befindliche Füllmasse, also die erste Komponente 8 ausgeschoben werden soll. Das Führungselement 11 ist vorgesehen, um den ersten Kolben 3 in der ersten Vorratskammer 6 zu führen.

Das Führungselement 11 umfasst einen Mischer 14, der insbesondere als statischer Mischer ausgebildet ist. Insbesondere ist das Führungselement 11 und der Mischer 14 als ein einziges Bauteil ausgeführt.

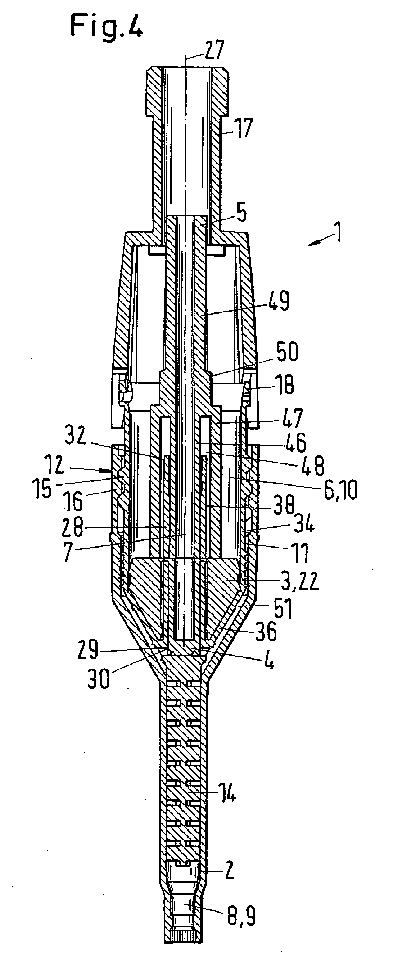

Der erste und zweite Kolben 3,4 sind mittels eines Stössels 5 bewegbar, um die beiden Komponenten 8,9 gleichzeitig auszutragen. Der Stössel 5 ist insbesondere derart ausgestaltet, dass er auf dem ersten und dem zweiten Kolben 3,4 aufliegt. Der Stössel 5 ist mit einem Gehäuseelement 17 einstückig verbunden solange sich die Mehrkomponentenkartusche im Lagerzustand befindet. Das Gehäuseelement 17 weist eine Sollbruchstelle 50 auf, über welche der Stössel 5 mit dem Gehäuseelement 17 im Lagerzustand verbunden ist. Diese Sollbruchstelle 50 wird zu Beginn des Austrags der Füllmasse durchtrennt, wie in Fig. 4 gezeigt ist. Der Stössel enthält zwei konzentrische Stösselkörper 46, 47, einen inneren Stösselkörper 46 und einen äusseren Stösselkörper 47. Der innere Stösselkörper 46 liegt auf dem zweiten Kolben 4 auf, der äussere Stösselkörper 47 liegt auf dem ersten Kolben 3 auf. Zwischen dem inneren und dem äusseren Stösselkörper ist eine ringförmige Ausnehmung 48 angeordnet, welche der Aufnahme der Trennwand 28 dient, wenn die Füllmasse aus der ersten und zweiten Vorratskammer 6,7 ausgetragen wird. Der innere Stösselkörper 46 und der äussere Stösselkörper 47 sind miteinander verbunden, sodass sie sich beim Austragvorgang gemeinsam bewegen, um die Kolben 3,4 in den entsprechenden Vorratskammern 6,7 zu verschieben. An die Stösselkörper schliesst ein Verbindungselement 49 an, welches derart ausgestaltet sein kann, dass es in ein handelsübliches Austraggerät eingepasst werden kann. Auch das Verbindungselement 49 ist innerhalb des Gehäuseelements 17 angeordnet. Das Verbindungselement 49 kann einen Hohlraum 50 umfassen, welcher der Einsparung von Material dient.

Das Führungselement 11 ist mittels eines Eingriffselements 18 mit einem Gehäuseelement 17 verbindbar.

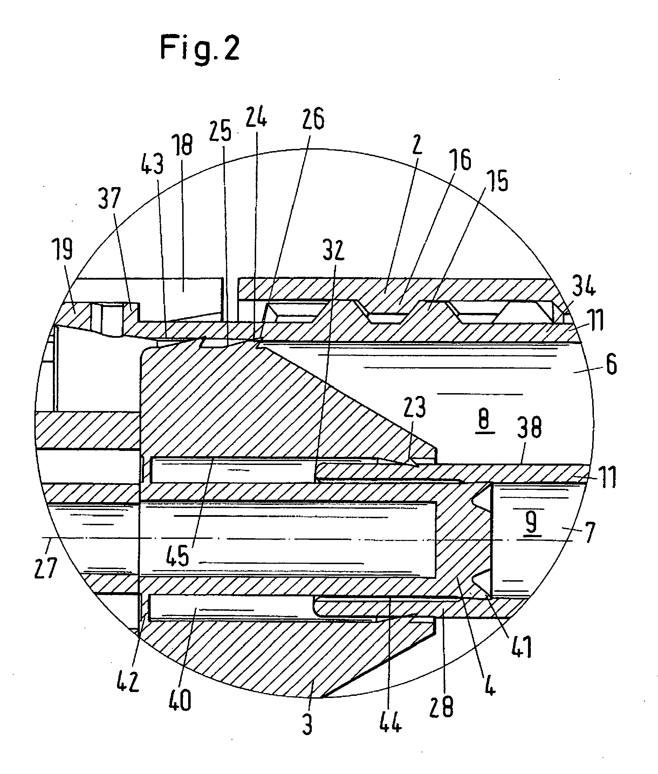

Der erste und der zweite Kolben 3,4 können miteinander verbunden sein, wie in Fig. 2 gezeigt ist. Insbesondere können sie als ein einziges Kolbenbauteil 39 ausgebildet sein. Das Kolbenbauteil 39 weist einen Schlitz 40 auf, der zur Aufnahme der Trennwand 28 des Führungselements 11 dient. Auf der Innenseite des Schlitzes schliesst der Kolben 4 an. Der Kolben 4 weist mindestens ein Dichtungselement 41 auf, welches insbesondere als Dichtungslippe ausgebildet ist. Ein Vorteil der Verwendung eines Kolbenbauteils 39 liegt darin begründet, dass das Kolbenbauteil kippsicher geführt werden kann. Zum einen greift in den Schlitz 40 das zweite Ende 32 der Trennwand 28 ein, zum anderen wird der äussere Kolbenmantel 25 entlang des Mantels 34 des Führungselements 11 geführt. Der äussere Kolbenmantel 25 weist zumindest eine ringförmige Dichtung 24 auf, der innere Kolbenmantel 45 weist ebenfalls zumindest eine ringförmige Dichtung 23 auf.

Der Schlitz 40 ist insbesondere ringförmig und weist am Schlitzgrund ein Brückenelement 42 auf, welches die Verbindung zwischen dem Kolben 3 und dem Kolben 4 des Kolbenbauteils 39 darstellt. Wird das Kolbenbauteil 39 zum Austrag der Füllmasse in Richtung der Austrittsöffnung 13 bewegt, also in der Fig. 2 nach rechts bewegt, dann wird das Brückenelement 42 durchtrennt, wenn es auf das zweite Ende 32 der Trennwand 28 auftrifft. Im Anschluss daran bewegen sich der Kolben 4 und der Kolben 3, der als Ringkolben 22 ausgebildet ist, parallel zueinander aber vollständig voneinander durch die Trennwand 28 getrennt. Die ringförmige Dichtung 23,24 kann ein Entlüftungselement 26 umfassen. Alternativ dazu kann ein Entlüftungselement 43, 44 am Führungselement 11, insbesondere am Mantelelement 34 und/oder auf der Trennwand 28 angebracht sein. Das Entlüftungselement 43 ist bevorzugt in der Nähe des zweiten Endbereichs 37 des Mantelelements 34 angebracht. Das Entlüftungselement 44 ist bevorzugt in der Nähe des zweiten Endes 32 der Trennwand 28 angebracht.

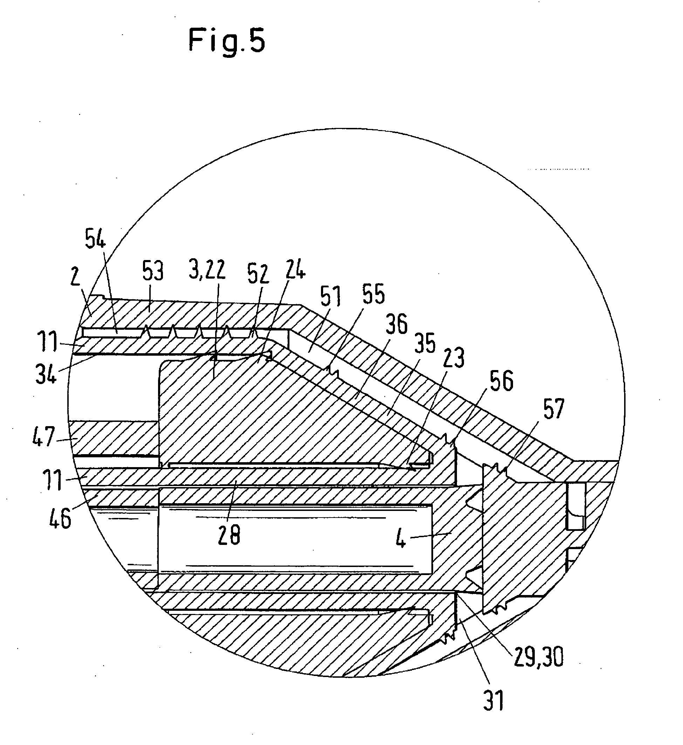

Fig. 3 zeigt ein Detail der Mehrkomponentenkartusche, welches den Bereich der ersten und zweiten Austrittsöffnungen 13, 29 umfasst. Das Führungselement 11 enthält eine Austrittsöffnung 13, durch welche die erste Komponente 8 aus der ersten Vorratskammer 6 austreten kann und das Führungselement 11 in einem Gehäuse 2 angeordnet ist, wobei das Führungselement 11 relativ zu dem Gehäuse 2 mittels eines Bewegungselements 12 bewegbar ist, wodurch die Austrittsöffnung 13 freigebbar ist. Das Bewegungselement 12 erlaubt eine relative Bewegung von Gehäuse 2 und Führungselement 11. Das Bewegungselement 12 umfasst gemäss einer bevorzugten Variante, die in Fig. 2 dargestellt ist, ein an dem Führungselement 11 angebrachtes Aussengewinde 15, in welches ein am Gehäuse 2 angebrachtes Innengewinde 16 eingreifen kann. Durch Betätigung des Bewegungselements 12, das heisst durch Drehung des Gehäuses 2 relativ zum Führungselement 11 verschiebt sich das Führungselement relativ zum Gehäuse derart, dass der erste Endbereich 35 des Mantelelements 34 einen Abstand zum Gehäuse 2 ausbildet. Hiermit wird die erste Austrittsöffnung 13 geöffnet, das heisst, die in der ersten Vorratskammer 6 befindliche Komponente 6 der Füllmasse kann durch die erste Austrittsöffnung 13 austreten und in dem zwischen Gehäuse 2 und erstem Endbereich 35 ausgebildeten Kanal in Richtung des Mischers 14 geführt werden. Im Bereich der zweiten Austrittsöffnung 29 kommt die erste Komponente 8 in Kontakt mit der zweiten Komponente 9, welche von der zweiten Vorratskammer 7 herkommend durch die Austrittsöffnung 29 austritt. Dieser Zustand ist auch in Fig. 5 dargestellt.

Fig. 4 zeigt die Mehrkomponentenkartusche nach Fig. 1 zu Ende des Austrags der Füllmasse aus der ersten und zweiten Vorratskammer 6,7. Der Stössel 5 wird relativ zum Gehäuseelement 17 bewegt, wenn der Stössel 5 mit einer Kraft beaufschlagt wird. Diese Kraft kann durch ein handelsübliches Austraggerät aber auch manuell aufgebracht werden. Die Verbindung zwischen Gehäuseelement 17 und Stössel 5, welche als Sollbruchstelle 50 ausgebildet ist, wird unterbrochen, wenn auf das Verbindungselement 49 eine Druckkraft ausgeübt wird.

Fig. 5 zeigt ein Detail der Mehrkomponentenkartusche gemäss Fig. 1 im Bereich der ersten Austrittsöffnung 13 in der Austragstellung. In Fig. 5 ist somit die Lage des Führungselements 11 relativ zum Gehäuse 2 dargestellt, wenn die Füllmasse aus den ersten und zweiten Vorratskammern 6,7 über den Mischer 14 ausgetragen worden ist, der Austrag also beendet ist. Das Füllmaterial hat somit als das den Mischer 14 verlassende Gemisch der ersten Komponente 8 und der zweiten Komponente 9 seine bestimmungsgemässe Verwendung gefunden. Bevor mit dem Austrag begonnen werden kann, also die Situation gemäss Fig. 3 vorliegt, muss das Bewegungselement 12, welches in Zusammenhang mit Fig. 2 bereits beschrieben wurde, betätigt werden. Durch Betätigung des Bewegungselements 12 wird die Austrittsöffnung 13 geöffnet.

Gemäss des vorliegenden bevorzugten Ausführungsbeispiels bildet sich durch Drehung des Bewegungselements 12 der in Zusammenhang mit Fig. 3 bereits erwähnte Kanal 51 zwischen dem Führungselement 11 und dem Gehäuse 2 aus, durch den die erste Komponente 8 durch die Austrittsöffnung oder eine Mehrzahl an Austrittsöffnungen 13 zum Mischer 14 gefördert wird. Die Austrittsöffnungen sind in der konischen Wand des ersten Endbereichs 35 des Führungselements 11 angebracht, wobei in Fig. 5 der Schnitt derart gelegt ist, dass der Verbindungssteg 36 gezeigt ist, der die Trennwand 28 des Führungselements 11 mit dem Mantelelement 34 des Führungselements 11 verbindet.

Die Austrittsöffnung 13 ist in der Darstellung gemäss Fig. 5 durch den Ringkolben 22 verschlossen, welcher den ersten Kolben 3 bildet. Die Austrittsöffnung 29 ist durch den zweiten Kolben 4 verschlossen. Die erste Komponente 8, welche sich in dem Kanal 51 befindet, kann nur in Richtung des Mischers 14 gefördert werden, da zwischen dem Mantelelement 34 des Führungselements 11 und dem Gehäuse 2 zumindest ein Dichtelement 52 angeordnet ist. Das Mantelelement 34 ist vorzugsweise zylinderförmig und konzentrisch zu dem Gehäuse 2 angeordnet, welches gleichfalls einen zylinderförmigen Abschnitt 52 hat. Der zwischen dem Mantelelement 34 und dem zylinderförmigen Abschnitt 52 bestehende Spalt 54 wird durch die Verschiebung des Gehäuses 2 relativ zum Führungselement 11 in seiner Breite nicht verändert, sodass die Abdichtung dieses Spalts 54 keine besonderen Probleme aufwirft.

Des weiteren zeigt Fig. 5 Dichtelemente 55, 56, 57 auf der Aussenseite des zweiten Endbereichs 36 des Mantelelements 34. Diese Dichtelemente 55, 56, 57 spielen bei der Befüllung der ersten und der zweiten Vorratskammern 6, 7 mit den entsprechenden ersten und zweiten Komponenten 8, 9 eine Rolle. Wenn die Vorratskammern 6,7 befüllt werden, liegt das Führungselement 11 an der Innenwand des Gehäuses 2 an. Der Spalt 51 hat idealerweise die Spaltbreite null. Bedingt durch Herstellungstoleranzen kann die Spaltbreite lokal grösser als null sein, daher werden die Dichtelemente 55 und 56 vorgesehen, um zu verhindern, dass in der Austrittsöffnung 13 befindliche erste Komponente 8 in einen derartigen schmalen Spalt gelangen kann. Die Dichtelemente 56 und 57 verhindern, dass die zweite Komponente 9 über die Auslassöffnung 29 in einen schmalen Spalt 51 gelangen kann. Durch die Dichtelemente wird somit vermieden, dass die erste und zweite Komponente in den Spalt gelangen und dort in unerwünschter Weise in Kontakt kommen.

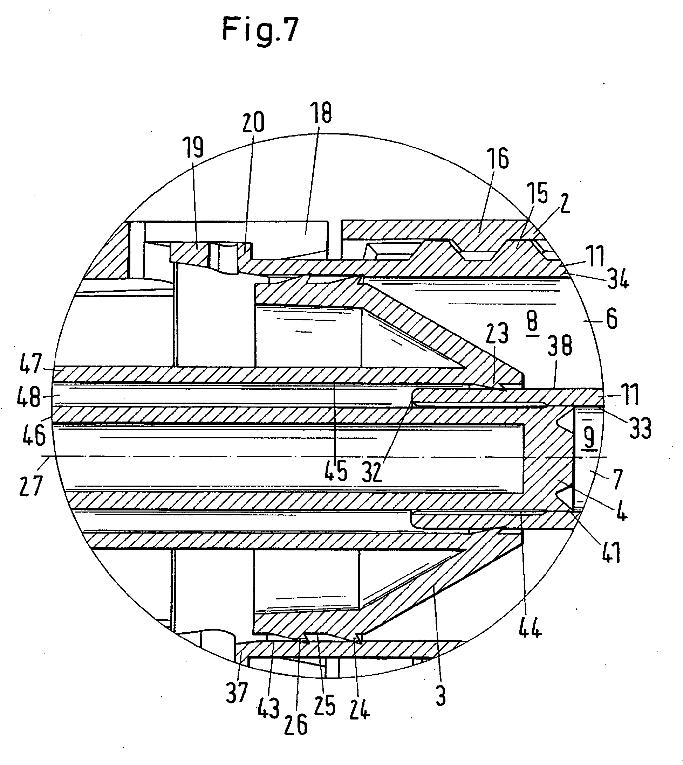

Fig. 5 wie auch Fig. 6 oder Fig. 7 zeigen des weiteren, dass das Gehäuse 2 mittels eines Eingriffselements 18 mit einem Gehäuseelement 17 verbindbar ist. Das Eingriffselement 18 umfasst ein Federelement 19. Das Federelement 19 ist als Vorsprung 20 am Umfang des Gehäuses 2 ausgebildet. Das Federelement 19 greift in eine Ausnehmung 21 des Gehäuseelements 17 ein, sodass das Gehäuseelement 17 drehfest mit dem Gehäuse 2 verbunden ist.

Fig. 6 zeigt eine Mehrkomponentenkartusche nach einem zweiten Ausführungsbeispiel, gemäss welchem der erste Kolben 3 und der Stössel 5 einstückig ausgebildet sind. Der Aufbau und die Funktionsweise dieser Mehrkomponentenkartusche unterscheidet sich ansonsten nicht vom ersten Ausführungsbeispiel, somit soll hier im wesentlichen auf die Beschreibung der Fig. 1 bis 5 verwiesen werden.

Ein wesentlicher Unterschied zu dem vorhergehenden Ausführungsbeispiel besteht darin, dass die Kolben 3,4 einstückig mit dem Stössel 5 ausgebildet sind. Da der Stössel ebenfalls mit dem Gehäuseelement 17 einstückig verbunden ist, wird die Anzahl Bauteile gegenüber dem vorherigen Ausführungsbeispiel um mindestens ein Bauteil reduziert. Die Kolben 3,4 sind zumindest teilweise hohl oder dünnwandig ausgeführt, was neben des verminderten Materialverbrauchs Vorteile in der Herstellung der Kolben haben kann.

Fig. 7 zeigt ein Detail des Kolbens der Mehrkomponentenkartusche gemäss Fig. 6. Fig. 7 ist die zu Fig. 2 korrespondierende Abbildung in welcher die unterschiedliche Gestaltung des Kolbens 3 zu sehen ist. Der Kolben 3 bildet zusammen mit dem Kolben 4 ein Kolbenbauteil 39, die durch die Ausnehmung 48 voneinander getrennt sind. Die Ausnehmung 48 dient der Aufnahme der Trennwand 28. In Bezug auf die Darstellung des Bereichs der Austragöffnungen wird auf Fig. 3 verwiesen. In Fig. 7 ist auch das Entlüftungselement 43 gezeigt, sowie das Entlüftungselement 44 an der Innenseite der Trennwand 33. Das Entlüftungselement hat bevorzugt die Form von zumindest einer nutförmigen Vertiefung in der Innenseite der Trennwand. Besonders bevorzugt ist eine Mehrzahl von Entlüftungselementen 44 symmetrisch zueinander angeordnet, in Fig. 7 sind 4 Entlüftungselemente 44 symmetrisch zueinander angeordnet.

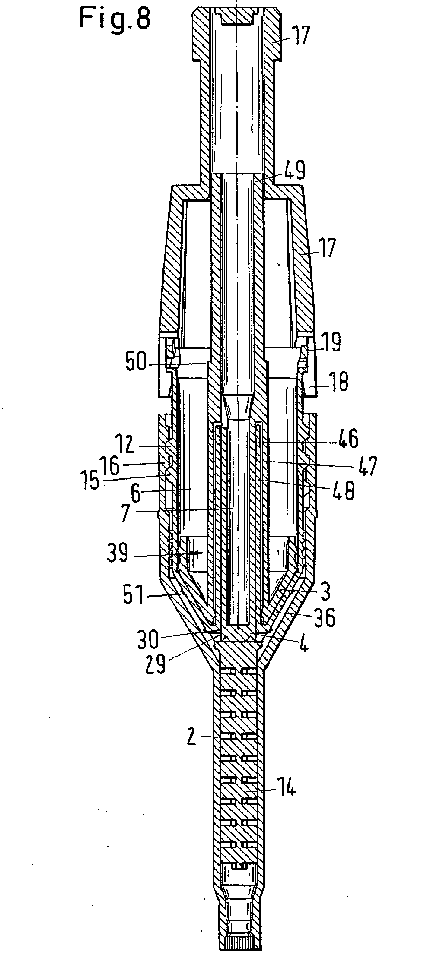

Fig. 8 zeigt die Mehrkomponentenkartusche gemäss Fig. 6 in der Austragstellung. Es wird auf die Beschreibung zu Fig. 6 verwiesen.

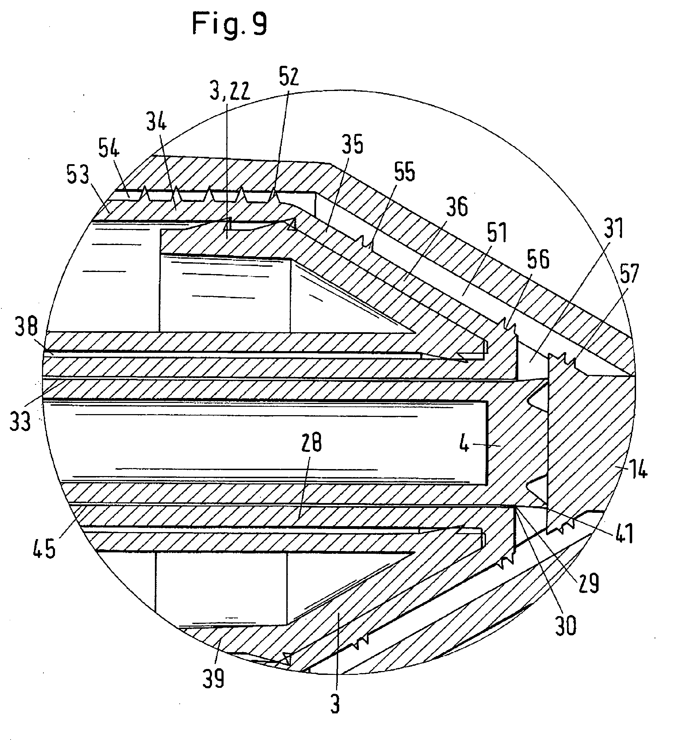

Fig. 9 zeigt ein Detail der Mehrkomponentenkartusche gemäss Fig. 6 im Bereich der Austrittsöffnung in Austragstellung. Die Funktionsweise entspricht ebenfalls der Funktionsweise gemäss Fig. 5, sodass auf die dortige Beschreibung verwiesen wird.

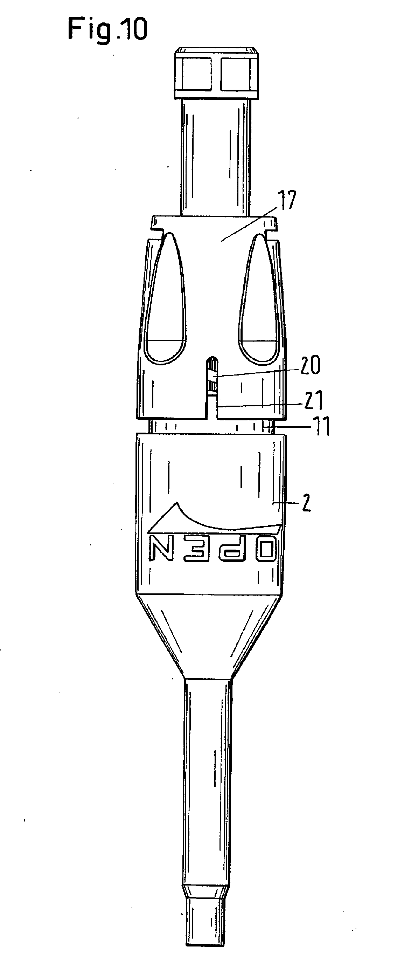

Fig. 10 zeigt die Mehrkomponentenkartusche nach einem der vorhergehenden Ausführungsbeispiele in einer Ansicht von aussen. Das Führungselement 11 ist mittels eines Eingriffselements 18 mit einem Gehäuseelement 17 verbindbar. Das Eingriffselement 18 kann ein Federelement 19 umfassen. Das Federelement 19 kann als Vorsprung 20 am Umfang des Führungselements 11 ausgebildet sein.

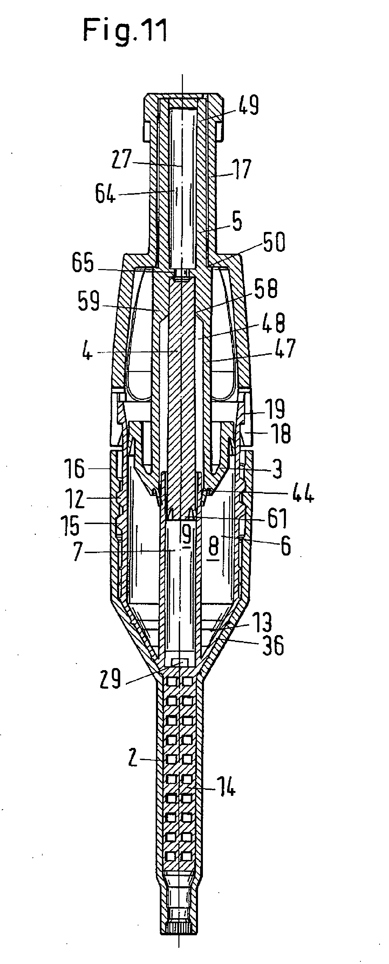

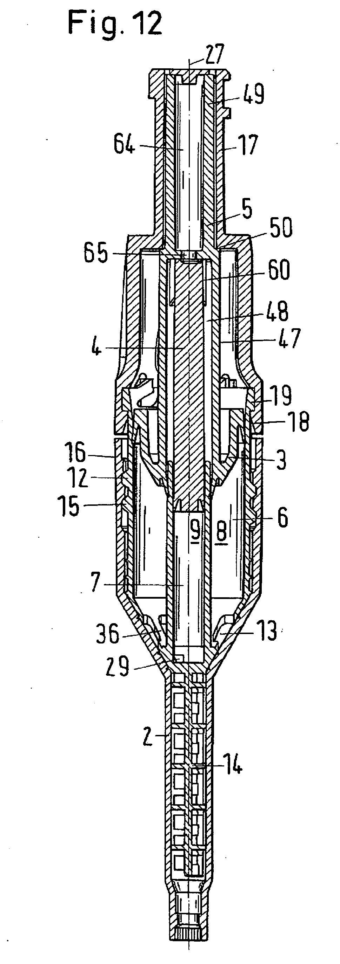

Fig. 11 und Fig. 12 zeigen eine weitere Variante der Mehrkomponentenkartusche. Wiederum sind gleiche Teile mit den gleichen Bezugszeichen versehen, sodass auf die entsprechende Beschreibung in den vorhergehenden Ausführungsbeispielen verwiesen wird. Fig. 11 zeigt somit einen Längsschnitt durch die Mehrkomponentenkartusche. Der erste und zweite Kolben 3,4 sind mittels eines Stössels 5 bewegbar, um die beiden Komponenten 8,9 gleichzeitig auszutragen. Der Stössel 5 ist insbesondere derart ausgestaltet, dass er mit dem ersten Kolben einstückig ausgebildet ist und der zweite Kolben 4 in dem Stössel 5 aufgenommen ist. Der Stössel 5 ist mit einem Gehäuseelement 17 einstückig verbunden solange sich die Mehrkomponentenkartusche im Lagerzustand befindet. Der zweite Kolben 4 wird insbesondere durch eine Steckverbindung 58 im Stössel 5 gehalten. Alternativ könnte auch eine Schraubverbindung oder eine Rastverbindung hierfür vorgesehen werden, das heisst eine Verbindung, mittels welcher der zweite Kolben 4 formschlüssig oder kraftschlüssig im Stössel gehalten ist. Insbesondere umfasst die Steckverbindung 58 zumindest ein Halteelement 59, bevorzugt eine Mehrzahl von Halteelementen, die als Halterippen ausgebildet sind. Besonders bevorzugt sind 4 Halterippen vorgesehen, mit welchen der zweite Kolben 4 gehalten und zentriert wird. Die Innenkante oder Innenfläche der Halterippen kann konisch ausgebildet sein, sodass der zweite Kolben 4 eingepasst werden kann. Der zweite Kolben 4 übernimmt gleichzeitig die Funktion eines Stössels. Das medienseitige erste Ende 61 des zweiten Kolbens 4 ist in der Trennwand 28 geführt. Der zweite Kolben 4 kann, wie gezeigt, als Vollkörper ausgeführt sein, aber auch zur Einsparung von Material und Gewicht zumindest teilweise als Hohlkörper ausgebildet sein.

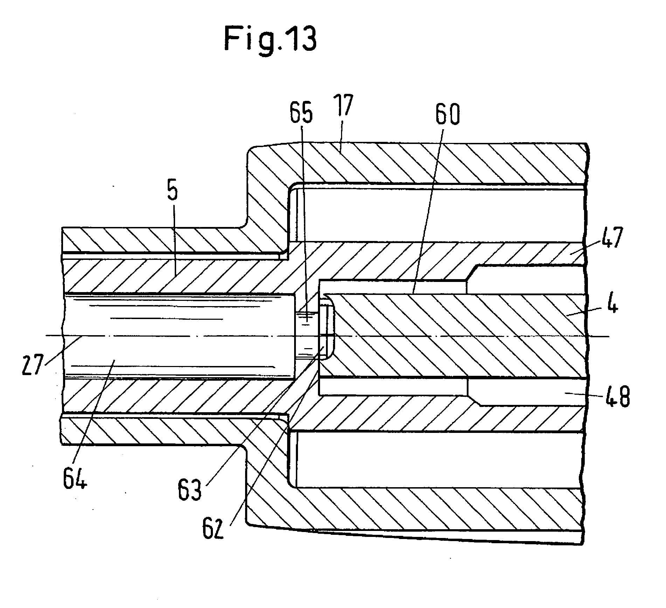

Fig. 12 zeigt dabei einen Längsschnitt, der gegenüber dem Längsschnitt der Fig. 11 entlang einer Ebene gelegt ist, die in Bezug auf die Schnittebene der Fig. 12 um 45° gedreht ist. In Fig. 12 ist gezeigt, dass zwischen den Halteelementen 59 ein Zwischenraum 60 liegt, der in Fig. 13 noch detaillierter dargestellt ist.

Fig. 13 zeigt im Detail das dem medienseitigen Ende 61 gegenüberliegende zweite Ende 62 des zweiten Kolbens 4. Diese zweite Ende 62 ist mit der vorhin beschriebenen Steckverbindung 58 in dem Stössel 5 gehalten. Der Zwischenraum 60 oder jeder der Zwischenräume, wenn eine Mehrzahl an Zwischenräumen vorgesehen ist, mündet in eine Aussparung 63. Durch die Aussparung 63 kann Gas, insbesondere Luft aus der zweiten Vorratskammer 7 über das Entlüftungselement 44 aus der Ausnehmung 48 durch eine Bohrung 65 in einen Kanal 64 im Inneren des Stössels geführt werden. Der Kanal 64 kann zur Atmosphäre offen sein, wie in Fig. 1 dargestellt, oder mit einem Verschlusselement versehen sein, wie in Fig. 11 oder Fig. 12 dargestellt. Die Aussparung 63 kann eine oder mehrere Nuten umfassen, insbesondere drei Nuten, die in einem Winkel von 120° zueinander angeordnet sind. Mittels der oder den Aussparungen 63 in Verbindung mit den Zwischenräumen 60 kann somit eine Entlüftung der Ausnehmung 48 erfolgen, welche den Zwischenraum zwischen dem mit dem ersten Kolben 3 verbundenen äusseren Stösselkörper 47 und dem zweiten Kolben 4 ausbildet.

## Patentansprüche

1. Mehrkomponentenkartusche (1), welche zur einmaligen Verwendung bestimmt ist, umfassend eine erste Vorratskammer (6) für eine erste Komponente (8), eine zweite Vorratskammer (7) für eine zweite Komponente (9), wobei die erste Vorratskammer (6) getrennt von der zweiten Vorratskammer (7) ist, wobei die erste Vorratskammer (6) koaxial um die zweite Vorratskammer (7) herum angeordnet ist und einen Ringraum (10) ausbildet, wobei in der ersten Vorratskammer (6) ein erster Kolben (3) beweglich aufgenommen ist und in der zweiten Vorratskammer (7) ein zweiter Kolben (4) beweglich aufgenommen ist, wobei der erste und zweite Kolben (3,4) mittels eines Stössels (5) bewegbar sind, um die beiden Komponenten (8.9) gleichzeitig auszutragen, wobei ein Führungselement (11) vorgesehen ist, um den ersten Kolben (3) in der ersten Vorratskammer (6) zu führen und um den zweiten Kolben (4) in der zweiten Vorratskammer (7) zu führen, **dadurch gekennzeichnet, dass** das Führungselement (11) eine Austrittsöffnung (13) enthält, durch welche die erste Komponente (8) aus der ersten Vorratskammer (6) austreten kann und das Führungselement (11) in einem Gehäuse (2) angeordnet ist, wobei das Führungselement (11) relativ zu dem Gehäuse (2) mittels eines Bewegungselements (12) bewegbar ist, wodurch die Austrittsöffnung (13) freigebbar ist.

2. Mehrkomponentenkartusche nach Anspruch 1, wobei das Führungselement (11) einen Mischer (14) beinhaltet.

3. Mehrkomponentenkartusche nach Anspruch 2, wobei der Mischer (14) als statischer Mischer ausgebildet ist.

4. Mehrkomponentenkartusche nach einem der vorhergehenden Ansprüche wobei das Bewegungselement (12) ein an dem Führungselement (11) angebrachtes Aussengewinde (15) umfasst, in welches ein am Gehäuse (2) angebrachtes Innengewinde (16) eingreifen kann.

5. Mehrkomponentenkartusche nach einem der vorhergehenden Ansprüche, wobei der erste Kolben (3) und der Stössel (5) einstückig ausgebildet sind.

6. Mehrkomponentenkartusche nach einem der vorhergehenden Ansprüche, wobei der Stössel (5) mit einem Gehäuseelement (17) einstückig verbunden ist.

7. Mehrkomponentenkartusche nach Anspruch 6, wobei das Gehäuseelement (17) eine Sollbruchstelle (50) aufweist, über welche der Stössel (5) mit dem Gehäuseelement (17) im Lagerzustand verbunden ist.

8. Mehrkomponentenkartusche nach Anspruch 7, wobei der Stössel (5) relativ zum Gehäuseelement (17) bewegbar ist, wenn der Stössel (5) mit einer Kraft beaufschlagt wird, wenn ein Austrag der ersten und zweiten Komponente erfolgen soll, sodass die Verbindung zwischen Gehäuseelement (17) und Stössel (5) unterbrochen wird.

9. Mehrkomponentenkartusche nach einem der vorhergehenden Ansprüche, wobei das Gehäuse (2) mittels eines Eingriffselements (18) mit einem Gehäuseelement (17) verbindbar ist.

10. Mehrkomponentenkartusche nach Anspruch 9, wobei das Eingriffselement (18) ein Federelement (19) umfasst.

11. Mehrkomponentenkartusche nach Anspruch 10, wobei das Federelement (19) als Vorsprung (20) am Umfang des Führungselements (11) ausgebildet ist.

12. Mehrkomponentenkartusche nach Anspruch 11, wobei das Federelement 19 in eine Ausnehmung 21 des Gehäuseelements 17 eingreift, sodass das Gehäuseelement 17 in einer Drehrichtung drehfest mit dem Gehäuse 2 verbunden ist.

13. Mehrkomponentenkartusche nach einem der vorhergehenden Ansprüche, wobei der erste Kolben 3 einen Ringkolben 22 umfasst, welcher eine ringförmige Dichtung 24 an seinem äusseren Kolbenmantel 25 aufweist.

14. Mehrkomponentenkartusche nach einem der vorhergehenden Ansprüche, wobei das Führungselement (11) ein Entlüftungselement (43, 44) umfasst.

15. Mehrkomponentenkartusche nach einem der vorhergehenden Ansprüche, wobei der Stössel 5 und der Kolben 3,4 zumindest teilweise hohl sind.

## Claims

1. A multicomponent cartridge (1) which is designed for single use, includes a first supply chamber (6) for a first component (8), a second supply chamber (7) for a second component (9), wherein the first supply chamber (6) is separate from the second supply chamber (7), wherein the first supply chamber (6) is arranged coaxially around the second supply chamber (7) and forms a ring space (10), wherein a first piston (3) is movably arranged in the first supply chamber (6) and a second piston (4) is movably arranged in the second supply chamber (7), wherein the first and second pistons (3, 4) are movable by means of a plunger (5) to simultaneously discharge the two components (8, 9), wherein a guide element (11) is provided to guide the first piston (3) in the first supply chamber (6) and to guide the second piston (4) in the second supply chamber (7), **characterized in that** the guide element (11) contains a discharge opening (13) through which the first component (8) can be discharged out of the first supply chamber (6) and **in that** the guide element (11) is arranged in a housing (2) wherein the guide element (11) is movable relative to the housing (2) by means of a movement element (12) whereby the discharge opening (13) can be freed.

2. A multicomponent cartridge in accordance with claim 1, wherein the guide element (11) includes a mixer (14).

3. A multicomponent cartridge in accordance with claim 2, wherein the mixer (14) is formed as a static mixer.

4. A multicomponent cartridge in accordance with any one of the preceding claims, wherein the movement element (12) includes an external thread (15) provided at the guide element (11) into which an inner thread (16) provided at the housing (2) can engage.

5. A multicomponent cartridge in accordance with any one of the preceding claims, wherein the first piston (3) and the plunger (5) are formed as one piece.

6. A multicomponent cartridge in accordance with any one of the preceding claims, wherein the plunger (5) is connected to a housing element (17) in one piece.

7. A multicomponent cartridge in accordance with claim 6, wherein the housing element (17) has a predetermined breaking point (50) via which the plunger (5) is connected to the housing element (17) in a storage state.

8. A multicomponent cartridge in accordance with claim 7, wherein the plunger (5) is movable relative to the housing element (17) when a force is applied to the plunger (5), when a discharge of the first and the second component should be effected, so that the connection between the housing element (17) and the plunger (5) is broken.

9. A multicomponent cartridge in accordance with any one of the preceding claims, wherein the housing (2) is connectable to a housing element (17) by means of an engagement element (18).

10. A multicomponent cartridge in accordance with claim 9, wherein the engagement element (18) includes a spring element (19).

11. A multicomponent cartridge in accordance with claim 10, wherein the spring element (19) is formed as a projection (20) at the perimeter of the guide element (11).

12. A multicomponent cartridge in accordance with claim 11, wherein the spring element (19) engages into a recess (21) of the housing element (17) so that the housing element (17) is rotationally fixedly connected to the housing (2) in one rotational irection.

13. A multicomponent cartridge in accordance with any one of the preceding claims, wherein the first piston (3) includes a ring piston (22) which has a ring shaped seal (24) at its outer piston skirt (25).

14. A multicomponent cartridge in accordance with any one of the preceding claims, wherein the guide element (11) includes a vent element (43, 44).

15. A multicomponent cartridge in accordance with any one of the preceding claims, wherein the plunger (5) and the piston (3, 4) are at least partially hollow.

## Revendications

1. Cartouche multi-composants (1), qui est destinée à une utilisation unique, comprenant une première enceinte de stockage (6) pour un premier composant (8), une deuxième enceinte de stockage (7) pour un deuxième composant (9), où la première enceinte de stockage (6) est séparée de la deuxième enceinte de stockage (7), où la première enceinte de stockage (6) est disposée coaxialement autour de la deuxième enceinte de stockage (7) et forme un espace annulaire (10), où est reçu dans la première enceinte de stockage (6) un premier piston (3) d'une manière mobile, et dans la deuxième enceinte de stockage (7), un deuxième piston (4) est reçu d'une manière mobile, où les premier et deuxième pistons (3, 4) sont déplaçables au moyen d'un poussoir (5) pour évacuer les deux composants (8, 9) simultanément, où un élément de guidage (11) est prévu pour guider le premier piston (3) dans la première enceinte de stockage (6) et pour guider le deuxième piston (4) dans la deuxième enceinte de stockage (7), **caractérisée en ce que** l'élément de guidage (11) comporte une ouverture de sortie (13) par laquelle le premier composant (8) peut sortir de la première enceinte de stockage (6), et l'élément de guidage (11) est disposé dans un boîtier (2), où l'élément de guidage (11) est déplaçable relativement au boîtier (2) au moyen d'un élément de déplacement (12), moyennant quoi l'ouverture de sortie (13) peut être libérée.

2. Cartouche multi-composants selon la revendication 1, où l'élément de guidage (11) comporte un mélangeur (14).

3. Cartouche multi-composants selon la revendication 2, où le mélangeur (14) est réalisé comme mélangeur statique.

4. Cartouche multi-composants selon l'une des revendications précédentes, où l'élément de déplacement (12) comprend un filetage extérieur (15) disposé à l'élément de guidage (11) dans lequel peut s'engager un taraudage (16) réalisé au boîtier (2).

5. Cartouche multi-composants selon l'une des revendications précédentes, où le premier piston (3) et le poussoir (5) sont réalisés en une pièce.

6. Cartouche multi-composants selon l'une des revendications précédentes, où le poussoir (5) est relié en une pièce à un élément de boîtier (17).

7. Cartouche multi-composants selon la revendication 6, où l'élément de boîtier (17) présente un emplacement de rupture de consigne (50) par lequel le poussoir (5) est relié à l'élément de boîtier (17) à l'état de stockage.

8. Cartouche multi-composants selon la revendication 7, où le poussoir (5) est déplaçable relativement à l'élément de boîtier (17), lorsque le poussoir (5) est chargé par une force lorsqu'une évacuation des premier et deuxième composants doit avoir lieu de sorte que la liaison entre l'élément de boîtier (17) et le poussoir (5) est interrompue.

9. Cartouche multi-composants selon l'une des revendications précédentes, où le boîtier (2) peut être relié au moyen d'un élément d'engagement (18) à un élément de boîtier (17).

10. Cartouche multi-composants selon la revendication 9, où l'élément d'engagement (18) comporte un élément de ressort (19).

11. Cartouche multi-composants selon la revendication 10, où l'élément de ressort (19) est réalisé comme saillie (20) au pourtour de l'élément de guidage (11).

12. Cartouche multi-composants selon la revendication 11, où l'élément de ressort (19) s'engage dans un évidement (21) de l'élément de boîtier (17) de sorte que l'élément de boîtier (17) est relié dans une direction de rotation d'une manière non tournante au boîtier (2).

13. Cartouche multi-composants selon l'une des revendications précédentes, où le premier piston (3) comprend un piston annulaire (22) qui présente un joint d'étanchéité annulaire (24) à sa jupe de piston extérieure (25).

14. Cartouche multi-composants selon l'une des revendications précédentes, où l'élément de guidage (11) comprend un élément d'aération (43, 44).

15. Cartouche multi-composants selon l'une des revendications précédentes, où le poussoir (5) et le piston (3, 4) sont au moins partiellement creux.
